(19) Europäisches Patentamt
European Patent Office
Office européen des brevets

(11) **EP 2 704 754 B1**

(12) **EUROPÄISCHE PATENTSCHRIFT**

(45) Veröffentlichungstag und Bekanntmachung des Hinweises auf die Patenterteilung:
**22.03.2017 Patentblatt 2017/12**

(21) Anmeldenummer: **12717749.1**

(22) Anmeldetag: **30.04.2012**

(51) Int Cl.:
*A61L 15/26* (2006.01)     *C08G 18/08* (2006.01)
*C08G 18/12* (2006.01)     *C08G 18/28* (2006.01)
*C08G 18/44* (2006.01)     *C08G 18/48* (2006.01)
*C08G 18/72* (2006.01)     *C08G 18/73* (2006.01)
*C08G 18/79* (2006.01)     *C08G 18/75* (2006.01)
*A61L 15/42* (2006.01)     *C08G 18/10* (2006.01)
*B82Y 30/00* (2011.01)     *C08K 3/36* (2006.01)
*C08L 75/04* (2006.01)

(86) Internationale Anmeldenummer:
**PCT/EP2012/057932**

(87) Internationale Veröffentlichungsnummer:
**WO 2012/150224 (08.11.2012 Gazette 2012/45)**

(54) **HYDROPHILER POLYURETHAN-SCHAUM MIT GERINGER VOLUMENQUELLUNG**

HYDROPHILIC POLYURETHANE FOAM WITH LOW VOLUME SWELLING

MOUSSE DE POLYURÉTHANE HYDROPHILE AU GONFLEMENT VOLUMIQUE MINIME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.05.2011 EP 11164764**

(43) Veröffentlichungstag der Anmeldung:
**12.03.2014 Patentblatt 2014/11**

(73) Patentinhaber: **Covestro Deutschland AG**
**51373 Leverkusen (DE)**

(72) Erfinder:
• **NIESTEN, Meike**
**51061 Köln (DE)**

• **DÖRR, Sebastian**
**40593 Düsseldorf (DE)**
• **PLUG, Sascha**
**51375 Leverkusen (DE)**
• **SCHÖNBERGER, Jan**
**42781 Haan (DE)**

(74) Vertreter: **Levpat**
**c/o Covestro AG**
**Gebäude 4825**
**51365 Leverkusen (DE)**

(56) Entgegenhaltungen:
**EP-A2- 0 335 669**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine spezielle Zusammensetzungen, die insbesondere zur Herstellung von hydrophilen, aliphatischen Polyurethan-Schäumen verwendet werden kann. Weitere Gegenstände der Erfindung sind ein Verfahren zur Herstellung eines hydrophilen, aliphatischen Polyurethan-Schaums, basierend auf der erfindungsgemäßen Zusammensetzung, ein nach dem Verfahren erhältlicher Polyurethan-Schaum sowie eine den Polyurethan-Schaum umfassende Wundauflage.

In der europäischen Patentanmeldung EP 2 143 744 A1 sind hydrophile aliphatische Polyurethanschäume beschrieben, die durch Umsetzung monomerarmer Prepolymere mit Wasser erhältlich sind. Das Absorptionsvermögen dieser Schäume für Flüssigkeiten wie Wasser ist zwar hoch, allerdings kommt es dabei auch zu einer starken Volumenquellung, d.h. das Volumen der Schäume vergrößert sich bei der Flüssigkeitsaufnahme erheblich. Dies ist insbesondere bei der Verwendung der Schäume in Wundauflagen nachteilig, da die Volumenzunahme zu einer Verformung der Wundauflage führt. Als Ergebnis dieser Verformung kann es zu einer unerwünschten Drückausübung auf die Wunde kommen. Im schlimmsten Fall löst sich die Wundauflage sogar ganz ab.

Aufgabe der vorliegenden Erfindung war es daher eine Zusammensetzung zur Herstellung eines Polyurethan-Schaums bereit zu stellen, der ein hohes Absorptionsvermögen für Flüssigkeiten wie Wasser und gleichzeitig nur eine geringe Volumenquellung aufweist

Diese Aufgabe wird erfindungsgemäß durch eine Zusammensetzung umfassend

A) isocyanatfunktionelle Prepolymere erhältlich durch Umsetzung von

A1) aliphatischen Diisocyanaten mit

A2) di- bis hexafunktionellen Polyalkylenoxiden mit einem Ethylenoxidanteil von 50 bis 100 mol%, bezogen auf die Gesamtmenge der enthaltenen Oxyalkylengruppen,

B) eine wässrige Polyurethansuspensionen, eine wässrige Polyacrylatsuspensionen oder wässrige Kieselsole.

gelöst.

Es hat sich gezeigt, dass unter Verwendung der Zusammensetzung ein Polyurethan-Schaum mit hohem Absorptionsvermögen für Flüssigkeiten wie Wasser erhalten werden kann, der gleichzeitig nur eine geringe Volumenquellung aufweist.

Eine wässrige Suspension im Sinne der vorliegenden Erfindung ist ein heterogenes Gemisch eines partikulären Feststoffs (disperse Phase) in einer Wasser enthaltenden flüssigen Phase (Dispergiermedium), bei der der mittlere Durchmesser der dispersen Phase bestimmt durch Laser-Korrelationsspektroskopie 1 bis 1000 nm, bevorzugt 2 bis 800 nm, weiter bevorzugt 5 bis 500 nm und besonders bevorzugt 20 bis 400 nm beträgt. Ebenfalls bevorzugt ist, wenn das Dispergiermedium ausschließlich aus Wasser besteht.

Gemäß einer bevorzugten Ausführungsform der Erfindung weisen die aliphatischen Diisocyanate A1) eine Molmasse von 140 bis 278 g/mol auf.

Beispiele für geeignete aliphatische Diisocyanate A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind. Besonders bevorzugt sind Butylendiisocyanat, Hexamethylendiisocyanat, Isophorondiisocyanat, ganz besonders bevorzugt Hexamethylendiisocyanat und Isophorondiisocyanat.

Vorteilhaft ist auch, wenn die di- bis hexafunktionellen Polyalkylenoxide A2) eine OH-Zahl von 22,5 bis 112 aufweisen.

Die Polyalkylenoxide können insbesondere auch einen Ethylenoxidgehalt von 50 bis 100 mol%, bevorzugt von 60 bis 85 mol%, bezogen auf die Gesamtmenge der enthaltenen Oxyalkylengruppen, aufweisen.

Die Polyalkylenoxide besitzen typischerweise zahlenmittlere Molekulargewichte von 1000 bis 15000 g/mol, bevorzugt von 3000 bis 8500 g/mol.

Ferner besitzen die Polyalkylenoxide der Komponente A2) bevorzugt OH-Funktionalitäten von 2 bis 6, weiter bevorzugt von 3 bis 6, besonders bevorzugt von 3 bis 4.

Geeignete Polyalkylenoxide sind beispielsweise Copolymere aus Ethylenoxid und Propylenoxid.

Besonders bevorzugt ist auch, wenn die Polyalkylenoxide auf Polyolen oder Aminen gestartet sind. Geeignete Starter sind Glycerin, Trimethylolpropan (TMP), Sorbit, Pentaerythrit, Triethanolamin, Ammoniak oder Ethylendiamin.

Bei der Herstellung der isocyanatfunktionellen Prepolymere A) wird das Verhältnis der Polyalkylenoxide A2) zu den niedermolekularen, aliphatischen Diisocyanaten A1) typischerweise so eingestellt, dass auf 1 Mol OH-Gruppen des Polyalkylenoxide A2) 2 bis 20 Mol, bevorzugt mit 2 bis 10 Mol und besonders bevorzugt 5 bis 10 Mol NCO-Gruppen des niedermolekularen, aliphatischen Diisocyanats A1) kommen.

Die Herstellung der Prepolymere kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung der Polyalkylenoxide A2) mit den Diisocyanaten A1) zum Prepolymer A) erfolgt typischerweise bei 25 bis 140 °C, bevorzugt bei 60 bis 100 °C.

Wurde bei der Umsetzung ein Überschuss an Diisocyanat eingesetzt, kann der nicht umgesetzte Rest anschließend bevorzugt durch Dünnschichtdestillation entfernt werden.

Vor, während und nach der Umsetzung der Diisocyanate mit den Polyalkylenoxiden oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien wie Di-*tert*-butylkresol oder Tocopherol zugesetzt werden.

Bevorzugt weisen die eingesetzten Prepolymere A) einen Restmonomergehalt von unter 0,5 Gew.-%, bezogen auf das Prepolymer auf. Dieser Gehalt kann durch entsprechend gewählte Einsatzmengen der Diisocyanate A1) und der Polyalkylenoxide A2) erreicht werden. Bevorzugt ist jedoch der Einsatz des Diisocyanats A) im Überschuss und mit anschließender, bevorzugt destillativer, Abtrennung nicht umgesetzter Monomere.

Der NCO-Gehalt der isocyanatfunktionellen Prepolymere A) beträgt bevorzugt 1,5 bis 4,5 Gew.-%, besonders bevorzugt 1,5 bis 3,5 Gew.-% und ganz besonders bevorzugt 1,5 bis 3,0 Gew.-%.

Die wässrige Suspension B) kann eine organische oder eine anorganische Suspension sein, d.h. organische oder anorganische Feststoffpartikel als disperse Phase enthalten. Beispiele für derartige Suspensionen B) sind wässrige Polyurethansuspensionen, wässrige Polyacrylatsuspensionen und wässrige Kieselsole. Besonders bevorzugt sind jedoch wässrige Polyurethansuspensionen, die insbesondere anionisch hydrophiliert sein können.

Ganz besonders bevorzugt enthält die erfindungsgemäße Zusammensetzung eine wässrige, anionisch hydrophilierte Polyurethan-Suspension B), die erhältlich sind, indem

J) isocyanatfunktionelle Prepolymere aus

J1) organischen Polyisocyanaten

J2) polymeren Polyolen insbesondere mit zahlenmittleren Molekulargewichten von 400 bis 8000 g/mol und / oder OH-Funktionalitäten von 1,5 bis 6,

J3) gegebenenfalls hydroxyfunktionellen Verbindungen, insbesondere mit Molekulargewichten von 62 bis 399 g/mol und

J4) gegebenenfalls isocyanatreaktiven, anionischen oder potentiell anionischen und / oder gegebenenfalls nichtionischen Hydrophilierungsmitteln,

hergestellt,
die isocyanatfunktionellen Prepolymere J) durch vollständige oder teilweise Umsetzung ihrer freien NCO-Gruppen mit

K) isocyanatreaktiven, bevorzugt aminofunktionellen, anionischen oder potentiell anionischen Hydrophilierungsmitteln und / oder

K2) gegebenenfalls aminofunktionellen Verbindungen, insbesondere mit Molekulargewichten von 32 bis 400 g/mol,

kettenverlängert und die Prepolymere J) vor während oder nach der Kettenverlängerung in Wasser dispergiert werden, wobei gegebenenfalls enthaltene potentiell anionische Gruppen durch teilweise oder vollständige Umsetzung mit einem Neutralisationsmittel in die anionische Form überführt werden.

Um eine anionische Hydrophilierung zu erreichen sollten in J4) und/oder K1) Hydrophilierungsmittel eingesetzt werden, die wenigstens eine gegenüber NCO-Gruppen reaktive Gruppe wie Amino-, Hydroxy- oder Thiolgruppen und darüber hinaus -COO$^-$ oder -SO$_3^-$ oder -PO$_3^{2-}$als anionische bzw. deren ganz oder teilweise protonierte Säureformen als potentiell anionische Gruppen aufweisen.

Bevorzugte wässrige, anionische Polyurethan-Suspensionen B) haben einen niedrigen Grad an hydrophilen anionischen Gruppen, bevorzugt von 0,1 bis 15 Milliequivalenten pro 100 g Festharz.

Um eine gute Sedimentationsstabilität zu erreichen, liegt die zahlenmittlere Teilchengröße der speziellen Polyurethan-Suspensionen bevorzugt bei weniger als 750 nm, besonders bevorzugt bei weniger als 550 nm, bestimmt mittels Laserkorrelations-Spektroskopie.

Das Verhältnis von NCO-Gruppen der Verbindungen aus Komponente J1) zu NCO-reaktiven Gruppen wie Amino-, Hydroxy- oder Thiolgruppen der Verbindungen der Komponenten J2) bis J4) beträgt bei der Herstellung des NCO-funktionellen Prepolymers J) 1,05 bis 3,5, bevorzugt 1,2 bis 3,0 besonders bevorzugt 1,3 bis 2,5.

Die aminofunktionellen Verbindungen in Stufe K1) werden vorzugsweise in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 bis 125 %, besonders bevorzugt zwischen 60 bis 120 % beträgt.

Geeignete Polyisocyanate der Komponente J1) sind die dem Fachmann an sich bekannten aromatischen, araliphatischen, aliphatischen oder cycloaliphatischen Polyisocyanate einer NCO-Funktionalität von $\geq$ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4''-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion- und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in J1) 1,6-Hexamethylendiisocyanat, Isophorondiisocyanat, die isomeren Bis(4,4'-isocyanatocyclohexyl)methane, sowie deren Mischungen eingesetzt.

In J2) werden polymere Polyole mit einem zahlenmittleren Molekulargewicht Mn bevorzugt von 400 bis 8000 g/mol und besonders bevorzugt von 600 bis 3000 g/mol eingesetzt. Diese weisen bevorzugt eine OH-Funktionalität von 1,8 bis 3, besonders bevorzugt von 1,9 bis 2,1 auf.

Geeignete polymere Polyole sind die in der Polyurethanlacktechnologie an sich bekannten Polyesterpolyole, Polyacrylatpolyole, Polyurethanpolyole, Polycarbonatpolyoie, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in J2) einzeln oder in beliebigen Mischungen untereinander eingesetzt werden.

Beispiele für geeignete Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Für die Herstellung geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe.

Bevorzugt ist Caprolacton.

Ebenfalls können in J2) Hydroxylgruppen aufweisende Polycarbonate, bevorzugt Poiycarbonatdiole, besonders bevorzugt mit zahlenmittleren Molekulargewichten $M_n$ von 400 bis 8000 g/mol und ganz besonders bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich. Beispiele hierfür geeigneter Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol, insbesondere 1,6-Hexandiol und/oder Hexandiolderivate, bezogen auf die zugrunde liegenden Diole. Diese Hexandiolderivate basieren auf Hexandiol und weisen neben endständigen OH-Gruppen Ester- oder Ethergruppen auf. Derartige Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in J2) eingesetzt werden.

Die Hydroxylgruppen aufweisenden Polycarbonate sind bevorzugt linear gebaut.

Ebenfalls können in J2) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Polyurethan Suspensionen B) enthalten als Komponente J2) eine Mischung aus Polycarbonatpolyolen und Polytetramethylenglykolpolyolen, wobei in dieser Mischung der Anteil an Polycarbonatpolyolen bevorzugt 20 bis 80 Gew.-% und der Anteil an Polytetramethylenglykolpolyolen bevorzugt 80 bis 20 Gew.-% betragen kann. Weiter bevorzugt ist ein Anteil von 30 bis 75 Gew.-% an Polytetramethylenglykolpolyolen und ein Anteil von 25 bis 70 Gew.-% an Polycarbonatpolyolen. Ganz besonders bevorzugt ist ein Anteil von 35 bis 70 Gew.% an Polytetramethylenglykolpolyolen und ein Anteil von 30 bis 65 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente J2) mindestens 50 Gew.-% bevorzugt 60 Gew.-% und besonders bevorzugt mindestens 70 Gew.% beträgt.

In J3) können gegebenenfalls hydroxyfunktionelle Verbindungen wie Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie $\alpha$-Hydroxybutyl-$\epsilon$-hydroxycapronsäureester, ($\omega$-Hydroxyhexyl-$\gamma$-hydroxybuttersäureester, Adipinsäure-($\beta$-hy-droxyethyl)ester oder Terephthalsäurebis($\beta$-hydroxyethyl)ester.

Ferner können in J3) auch monofunktionelle, hydroxylgruppen-haltige Verbindungen eingesetzt werden. Beispiele solcher monofunktionellen Verbindungen sind Ethanol, n-Butanol, Ethylenglykolmonobutylether, Diethylenglykolmonomethylether, Ethylen-glykolmonobutylether, Diethylenglykolmonobutylether, Propylenglykolmonomethylether, Dipropylenglykol-monomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Besonders bevorzugte Verbindungen der Komponente J3) sind 1,6-Hexandiol. 1,4-Butandiol, Neopentylglykol und Trimethylolpropan.

Unter anionisch bzw. potentiell anionisch hydrophilierenden Verbindungen der Komponente J4) werden sämtliche Verbindungen verstanden, die mindestens eine isocyanatreaktive Gruppe wie eine Hydroxylgruppe sowie mindestens eine Funktionalität wie z.B. $-COO^-M^+$, $-SO_3^-M^+$, $-PO(O-M^+)_2$ mit $M^+$ beispielsweise gleich Metallkation, $H^+$, $NH_4^+$, $NHR_3^+$, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann, aufweisen. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und $NaHSO_3$, wie es in DE-A 2 446 440, Seite 5 - 9, Formel I-III beschrieben ist. Bevorzugte anionische oder potentiell anionische Hydrophilierungsmittel der Komponente J4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel der Komponente J4) sind solche, die Carboxylat- bzw Carbonsäuregruppen als ionische oder potentiell ionische Gruppen enthalten, wie Dimethylolpropionsäure, Dimethylolbuttersäue und Hydroxypivalinsäure bzw. deren Salze.

Geeignete nichtionisch hydrophilierende Verbindungen der Komponente J4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese Verbindungen sind entweder reine Polyethylenoxidether oder gemischte Polyalkylenoxidether, wobei sie dann bevorzugt mindestens 30 mol-% und weiter bevorzugt mindestens 40 mol-% bezogen auf alle enthaltenen Alkylenoxideinheiten an Ethylenoxideinheiten enthalten.

Ganz besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Unter anionisch bzw. potentiell anionisch hydrophilierenden Verbindungen der Komponente K) werden sämtliche Verbindungen verstanden, die mindestens eine isocyanatreaktive Gruppe, bevorzugt eine Amino-Gruppe aufweisen, sowie mindestens eine Funktionalität wie z.B.-$COO^-M^-$, -$SO_3^-M^+$, -$PO(O^-M^+)_2$ mit $M^+$ beispielsweise gleich Metallkation, $H^+$, $NH_4^+$, $NHR_3^+$, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen K1) sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Aminoethylamino)ethansulfonsäure, Ethylendiamin-propyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente K1) sind solche der vorstehend genannten Art, die über Carboxylat- bzw. Carobonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

Als Komponente K2) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, $\alpha,\alpha,\alpha',\alpha'$-Tetramethyl-1,3-und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin oder sowie Hydraziden wie Adipinsäuredihydrazid.

Darüber hinaus können als Komponente K2) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan 3-Amino-1- Methylaminobutan, Alkanolamine wie N-Aminoethylethanolamin, Ethanolamin, 3-Aminopropanol, Neopentanolamin.

Ferner können als Komponente K2) auch monofunktionelle isocyanatreaktive Aminverbindungen wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet werden,

Bevorzugte Verbindungen der Komponente K2) sind 1,2-Ethylendiamin, 1,4-Diaminobutan und Isophorondiamin.

In einer weiteren bevorzugten Ausführungsform werden die Komponenten J1 bis J4) und K1) bis K2) in den folgenden Mengen eingesetzt, wobei sich die Einzelmengen stets zu 100 Gew.-% addieren:

5 bis 40 Gew.-% Komponente J1),

55 bis 90 Gew.-% Komponente J2),

0,5 bis 20 Gew.-% Summe der Komponenten J3) und K1)

0,1 bis 25 Gew.-% Summe der Komponenten J4) und K2),

wobei bezogen auf die Gesamtmengen der Komponenten J1) bis J4) und K) bis K2) 0,1 bis 5 Gew.-% an anionischen bzw. potentiell anionischen Hydrophilierungsmitteln J4) und/oder K2) verwendet werden.

In einer besonders bevorzugten Ausführungsform der Polyurethan-Suspensionen B) werden die Komponenten J1) bis J4) und K) bis K2) in den folgenden Mengen eingesetzt, wobei sich die Einzelmengen stets zu 100 Gew.-% aufaddieren:

5 bis 35 Gew.-% Komponente J1),

60 bis 90 Gew.-% Komponente J2),

0,5 bis 15 Gew.-% Summe der Komponenten J3) und K1)

0,1 bis 15 Gew.-% Summe der Komponenten J4) und K2),

wobei bezogen auf die Gesamtmengen der Komponenten J1) bis J4) und K1) bis K2) 0,2 bis 4 Gew.-% an anionischen bzw. potentiell anionischen Hydrophilierungsmitteln J4) und/oder K2) verwendet werden.

In einer ganz besonders bevorzugten Ausführungsform der Polyurethan-Suspensionen werden die Komponenten J1) bis J4) und K1) bis K2) in den folgenden Mengen eingesetzt, wobei sich die Einzelmengen stets zu 100 Gew.-% aufaddieren:

10 bis 30 Gew.-% Komponente J1),

65 bis 85 Gew.-% Komponente J2),

0,5 bis 14 Gew.-% Summe der Komponenten J3) und K1)

0,1 bis 13,5 Gew.-% Summe der Komponenten J4) und K2),

wobei bezogen auf die Gesamtmengen der Komponenten J1) bis J4) und K1) bis K2) 0,5 bis 3,0 Gew.-% an anionischen bzw. potentiell anionischen Hydrophilierungsmitteln J4) und/oder K2) verwendet werden.

Die Herstellung der anionisch hydrophilierten Polyurethan-Suspensionen B) kann in einer oder mehreren Stufe/-n in homogener oder bei mehrstufiger Umsetzung, teilweise in disperser Phase durchgeführt werden. Nach vollständig oder teilweise durchgeführter Polyaddition von J1) bis J4) erfolgt ein Dispergier-, Emulgier- oder Lösungsschritt. Im Anschluss erfolgt gegebenenfalls eine weitere Polyaddition oder Modifikation in disperser oder gelöster (homogener) Phase.

Hierfür können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren gearbeitet.

Für die Herstellung nach dem Aceton-Verfahren werden üblicherweise die Bestandteile J2) bis J4) und die Polyisocyanatkomponente J1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C erhitzt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von, N-Methylpyrrolidon, N-Ethylpyrrolidon in der Suspension verbleiben. Bevorzugt werden aber außer den üblichen aliphatischen, ketofunktionellen Lösemitteln keine anderen Lösungsmittel verwendet.

Bei der Herstellung des Polyurethan-Prepolymeren aus J1) bis J4) kann das Stoffmengenverhältnis von Isocyanatgruppen zu mit isocyanatreaktiven Gruppen 1,05 bis 3,5, bevorzugt 1,2 bis 3,0, besonders bevorzugt 1,3 bis 2,5 betragen.

Die Umsetzung der Komponenten J1) bis J4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmo-noalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt insbesondere 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss kann in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung werden $NH_2$- und/oder NH-funktionelle Komponenten mit den noch verbliebenen Isocyanatgruppen des Prepolymers teilweise oder vollständig umgesetzt.

Bevorzugt wird die Kettenverlängerung/-terminierung vor der Dispergierung in Wasser durchgeführt.

Zur Kettenterminierung werden üblicherweise Amine K2) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionischen oder potentiell anionische Hydrophilierungsmittel entsprechend der Definition K1) mit $NH_2$- oder NH-Gruppen eingesetzt, erfolgt die Kettenverlängerung der Prepolymere bevorzugt vor der Dispergierung.

Die aminischen Komponenten K1) und K2) können gegebenenfalls in wasser- oder lösemittelverdünnter Form einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung bzw. Suspendierung erfolgt bevorzugt im Anschluss an die Kettenverlängerung. Dazu wird das gelöste und kettenverlängerte Polyurethan-Polymer gegebenenfalls unter starker Scherung, wie z.B. starkem Rühren, entweder in das Dispergierwasser eingetragen oder es wird umgekehrt das Suspendierwasser zu den kettenverlängerte Polyurethan-Polymerlösungen gerührt. Bevorzugt wird das Wasser in das gelöste kettenverlängerte Polyurethan-Polymer gegeben.

Das in den Suspensionen nach dem Dispergierschritt noch enthaltene organischen Lösemittel wird üblicherweise anschließend destillativ entfernt. Eine Entfernung bereits während der Dispergierung ist ebenfalls möglich.

Der Restgehalt an organischen Lösemitteln in den Polyurethan-Suspensionen B) beträgt typischerweise weniger als 1,0 Gew.-%, bevorzugt weniger als 0,5 Gew.-%, bezogen auf die gesamte Suspension.

Der pH-Wert der Polyurethan-Suspensionen B) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Suspensionen B) beträgt typischerweise 40 bis 70, bevorzugt 50 bis 65, besonders bevorzugt 55 bis 65 Gew.-%. Eine weitere Verdünnung der Suspension mit Wasser vor dem Einsatz ist ebenso möglich, wie die Verwendung der Suspension und Wasser als eigenständiger Bestandteil der Zusammensetzung.

Geeignete wässrige Kieselsole sind kolloidale Supensionen von amorphem Siliciumdioxid in Wasser, die auch als Siliciumdioxidsole meist aber kurz als Kieselsole bezeichnet werden. Das Siliciumdioxid liegt dabei in Form von kugelförmigen und an der Oberfläche hydroxylierten Partikeln vor. Der Partikeldurchmesser der Kolloidteilchen beträgt in der Regel 1 bis 200 nm.

Typische wässrige Polyacrylat-Suspension enthalten beispielsweise eine oder mehrere der folgenden Aufbaukomponenten:

a) Styrol und / oder andere vinylaromatische Verbindungen,

b) Acrylsäureester,

c) Polyvinylidenverbindung einer Funktionalität $\geq 2$,

d) säurefunktionelle, olefinisch ungesättigte Monomere,

e) Methacrylsäureester,

f) weitere olefinisch ungesättigte Verbindungen, die nicht in a) bis e) aufgeführt sind.

Geeignete vinylaromatische Verbindungen a) insbesondere mit bis zu 20 C-Atomen sind beispielsweise Styrol, Vinyltoluol, o- und p-Methylstyrol, Butylstyrol, Decylstyrol, halogenierte Styrole, wie beispielsweise Monochlorstyrole, Dichlorstyrole, Tribromstyrole oder Tetrabromstyrole. Bevorzugt ist Styrol.

Geeignete Acrylsäureester b) umfassen insbesondere Methylacrylat, Ethylacrylat, Propylacrylat, Isopropylacrylat, n-Butylacrylat, sec-Butylacrylat, tert-Butylacrylat, Pentylacrylat, Hexylacrylat, Heptylacrylat, Octylacrylat, 2-Octylacrylat, Ethylhexylacrylat, Nonylacrylat, 2-Methyl-octylacrylat, 2-tert.-Butylheptylacrylat, 3-iso-Propylheptyl-acrylat, Decylacrylat, Undecylacrylat, 5-Methylundecylacrylat, Dodecylacrylat, 2-Methyldodecylacrylat, Tridecylacrylat, 5-Methyltridecylacrylat, Tetradecylacrylat, Pentadecylacrylat, Hexadecylacrylat, 2-Methylhexade-cylacrylat, Heptadecylacrylat, 5-iso-Propylheptadecylacrylat, 5-Ethyloctadecylacrylat, Octadecylacrylat, Nona-decylacrylat, Eicosylacrylat, Cycloalkylacrylate, wie beispielsweise Cyclopentylacrylat, Cyclohexylacrylat, 3-Vinyl-2-butylcyclohexylacrylat, Cycloheptylacrylat, Cyclooctylacrylat, Bornylacrylat, Tetrahydrofurfurylacrylat und Isobornylacrylat. Bevorzugt sind Ethylacrylat, n-Butylacrylat, Ethylhexylacrylat, Cyclohexylacrylat, besonders bevorzugt sind Ethylacrylat, n-Butylacrylat oder Ethylhexylacrylat.

Zu den geeignete Polyvinylidenverbindungen c) zählen solche Verbindungen, die mindestens über zwei olefinisch ungesättigte Bindungen verfügen. Dazu zählen insbesondere Acryl- oder Methacrylsäurester von Polyolen einer Funktionalität $\geq 2$, wie z.B. Ethylenglykoldiacrylat, Diethylenglykoldiacrylat, Glycerindiacrylat, Glycerintriacrylat, Ethylenglykoldimethacrylat, 1,3-Propandioldiacrylat, 1,3-Propandioldimethacrylat, 1,4-Butandioldiacrylat, 1,4-Butandioldimethacrylat, 1,6-Hexandioldiacrylat, 1,6-Hexandioldimethacrylat, 1,2,4-Butantrioltrimethacrylat, 1,4-Cyclohexandioldiacrylat, 1,4-Benzoldioldimethacrylat, Pentaerythritoltri- und tetraacrylat bzw. -methacrylat, Dipentaerythritolhexaacrylat, Tripentaerythritolhexaacrylat, Tripentaerythritoloctacrylat, Trimethylolpropantriacrylat, Trimethylolpropantrimethacrylat, Trimethylolethantriacrylat, Sorbitolhexaacrylat, 1,3-Propandioldiacrylat, 1,5-Pentadioldimethacrylat, 1,9-Nonandioldimethacrylat, 1,10-Decandioldimethacrylat, Propylenglykoldiacrylat, Dipropylenglykoldiacrylat, Diacrylate und Dimethacrylate vom Polyethylenglykol einer Molmasse von 200 bis 1500 g/mol. Bevorzugt sind 1,4-Butandioldiacrylat, Trimethylolpropandimethacrylat, Ethylenglykoldimethacrylat, 1,6-Hexandioldimethacrylat, besonders bevorzugt sind Ethylenglykoldimethacrylat oder 1,6-Hexandioldimethacrylat. Es ist auch möglich Mischungen der entsprechenden polyfunktionellen Vernetzer zu verwenden.

Geeignete olefinisch ungesättigte, säurefunktionelle Monomere d) sind sulfon-, phosphat- oder carbonsäurefunktionelle Monomere, bevorzugt sind carbonsäurefunktionelle Monomere wie Acrylsäure, Methacrylsäure, β-Carboxyethylacrylat, Crotonsäure, Fumarsäure, Maleinsäureanhydrid, Itaconsäure oder Monoalkylester zweibasiger Säuren bzw. Anhydride wie z.B. Maleinsäure-monoalkylester. Ferner geeignet als Verbindungen der Komponente d) sind auch ungesättigte, radikalisch polymerisierbare Verbindungen mit Phosphat- bzw. Phosphonat- oder Sulfonsäure- bzw. Sulfonatgruppen, wie z.B. in der WO-A 00/39181 (S. 8, Z. 13 - S. 9, Z. 19) beschrieben werden. Besonders bevorzugt sind Acryl- oder Methacrylsäure, ganz besonders bevorzugt ist Acrylsäure.

Geeignete Ester e) der Methacrylsäure umfassen insbesondere Methylmethacrylat, Ethylmethacrylat, Propylmethacrylat, Isopropylmethacrylat, n-Butylmethacrylat, sec-Butylmethacrylat, tert-Butylmethacrylat, Pentylmethacrylat, Hexylmethacrylat, Heptylmethacrylat, Octylmethacrylat, 2-Octylmethacrylat, Ethylhexylmethacrylat, Nonylmethacrylat, 2-Methyloctylmethacrylat, 2-tert.-Butylheptylmethacrylat, 3-iso-Propylheptylmethacrylat, Decylmethacrylat, Undecylmethacrylat, 5-Methylundecylmethacrylat, Dodecylmethacrylat, 2-Methyldodecylmethacrylat, Tridecylmethacrylat, 5-Methyltridecylmethacrylat, Tetradecylmethacrylat, Pentadecylmethacrylat, Hexadecylmethacrylat, 2-Methylhexadecylmethacrylat, Heptadecylmethacrylat, 5-iso-Propylheptadecylme-thacrylat, 5-Ethyloctadecylmethacrylat, Octadecylmethacrylat, Nonadecylmethacrylat, Eicosylmethacrylat, Cycloalkylinethacrylate, wie beispielsweise Cyclopentylmethacrylat, Cyclohexylmethacrylat, 3-Vinyl-2-butylcyclohexylmethacrylat, Cycloheptylmethacrylat, Cyclooctylmethacrylat, Bornylmethacrylat, Tetrahydrofurfurylmethacrylat oder Isobornylmethacrylat. Weiterhin können die Methacrylsäurederivate auch in Form der korrespondierenden Nitrile oder Amide eingesetzt werden, wie z.B. Methacrylnitril oder Methacrylamid. Außerdem besteht die Möglichkeit andere funktionelle Monomere in Abhängigkeit von der gewünschten Anwendung zu verwenden, wie beispielsweise Diacetonmethacrylamid oder Acetoacetoxyethylmethacrylat. Bevorzugt sind Methylmethacrylat, Ethylmethacrylat, Butylmethacrylat, tert-Butylmethacrylat, besonders bevorzugt sind Methylmethacrylat, tert-Butylmethacrylat oder Butylmethacrylat.

Die Herstellung der erfindungsgemäßen Polyacrylat-Suspension kann auf an sich bekannte Weise, beispielsweise mittels Emulsionspolymerisation, erfolgen.

Die in den erfindungsgemäße Polyacrylat-Suspensionen enthaltenen Polymer-Partikel weisen in der Regel eine mittlere Teilchengröße von 20 bis 400 nm, bevorzugt von 90 bis 170 nm, besonders bevorzugt von 100 bis 130 nm auf.

Der Feststoffgehalt einer wässrigen Suspension wird generell über das Verhältnis von Wasser zu organischen Ausgangsstoffen bestimmt. Der Feststoffgehalt der erfindungsgemäßen Polyacrylat-Suspension liegt bevorzugt zwischen 5 und 65 Gew.-%, weiter bevorzugt zwischen 30 und 55 Gew.% und besonders bevorzugt zwischen 35 und 45 Gew.-%.

Die wässrige Suspension B) wird typischerweise in einer Menge 1 bis 50, bevorzugt 5 bis 30 und besonders bevorzugt 5 bis 25 Gew % bezogen auf Festgehalt Dispersion auf den Festgehalt des fmalen Schaums

Gemäß einer weiteren bevorzugten Ausführungsform kann die erfindungsgemäße Zusammensetzung

A) heterocyclische, 4-Ring- oder 6-Ring-Oligomere von niedermolekularen, aliphatischen Diisocyanaten mit einer Molmasse von 140 bis 278 g/mol umfassen.

Bespiele geeignete Oligomere C) sind heterocyclische, 4-Ring- oder 6-Ring-Oligomere von niedermolekularen, aliphatischen Diisocyanaten mit einer Molmasse von 140 bis 278 g/mol wie Isocyanurate, Iminooxadiazindione oder Uretdione der vorgenannten niedermolekularen, aliphatischen Diisocyanate. Bevorzugt sind heterocyclische 4-Ring-Oligomere wie Uretdione. Der durch den Einsatz der Komponente C) erhöhte Gehalt an Isocyanatgruppen sorgt für ein besseres Aufschäumen, da bei der Isocyanat-Wasser-Reaktion mehr $CO_2$ gebildet wird.

Die erfindungsgemäße Zusammensetzung kann auch

B) C8- bis C22-Monocarbonsäuren oder deren Ammonium- oder Alkalisalze oder C12- bis C44-Dicarbonsäuren oder deren Ammonium- oder Alkalisalze oder Alkalimetallsalze schwacher anorganischer Säuren umfassen.

Beispiele geeigneter Verbindungen der Komponente D) sind die Ammonium-, Na-, Li- oder K-Salze von Ethylhexansäure, Octansäure, Decansäure, Dodecansäure, Palmitinsäure, Stearinsäure, den Octadecensäuren, den Octadecadiensäuren, den Octadecatriensäuren, Isostearinsäure, Erucasäure, Abietinsäure und ihren Hydrierungsprodukten. Beispiele für $C_{12}$- bis $C_{44}$-Dicarbonsäuren bzw. die daraus abgeleiteten Ammonium- und Alkalisalze sind Dodecandisäure, Dodecenyl-, Tetradecenyl-, Hexadecenyl- und Octadecenyl-Bernsteinsäure, $C_{36}$- und $C_{44}$-Dimerfettsäuren und ihre Hydrierungsprodukte sowie die entsprechenden Ammonium-, Na-, Li- oder K-Salze dieser Dicarbonsäuren.

Alkalimetallsalze schwacher anorganischer Säuren im Sinne der Erfindung sind definiert dadurch, dass deren korrespondierende freie Säuren in Wasser bei 25 °C einen pKs-Wert von $\geq 4{,}0$ und $\leq 14{,}0$ aufweisen. Beispiele sind Kaliumhydroxid, Natriumhydroxid, Kaliumcarbonat, Natriumcarbonat und Natriumhydrogencarbonat, wobei auch beliebige Mischungen dieser Salze geeignet sind.

Gemäß noch einer weiteren bevorzugten Ausführungsform kann die erfindungsgemäße Zusammensetzung

C) hydrophile Polyisocyanate, erhältlich durch Umsetzung von

E1) aliphatischen Diisocyanaten insbesondere einer Molmasse von 140 bis 278 g/mol und / oder daraus herstellbaren Polyisocyanaten mit einer Isocyanatfunktionalität von 2 bis 6 mit

E2) monofunktionellen Polyalkylenoxiden insbesondere einer OH-Zahl von 10 bis 250 und einem Ethylenoxidanteil von 50 bis 100 mol%, bezogen auf die Gesamtmenge der enthaltenen Oxyalkylengruppen,

umfassen.

Bei der Herstellung der hydrophilen Polyisocyanate E) kann das Verhältnis der monofunktionellen Polyalkylenoxide E2) zu den aliphatischen Diisocyanaten E1) so eingestellt werden, dass auf 1 Mol OH-Gruppen der monofunktionellen Polyalkylenoxide 1,25 bis 15 Mol, bevorzugt 2 bis 10 Mol und besonders bevorzugt 2 bis 6 Mol NCO-Gruppen des aliphatischen Diisocyanats E1) kommen. Anschließend erfolgt dann bevorzugt die Allophanatisierung bzw. Biurethisierung und/oder Isocyanuratbildung bzw. Uretdionbildung. Werden die Polyalkylenoxide E2) über Urethangruppen an die aliphatischen Diisocyanate E1) gebunden, findet bevorzugt im Anschluss eine Allophanatisierung statt. Weiterhin ist bevorzugt, dass Isocyanurat-Struktureinheiten gebildet werden.

Eine alternative Herstellung der hydrophilen Polyisocyanate E) erfolgt typischerweise durch Umsetzung von 1 Mol OH-Gruppen der monofunktionellen Polyalkylenoxidkomponente E2) mit 1,25 bis 15 Mol, bevorzugt 2 bis 10 Mol und besonders bevorzugt 2 bis 6 Mol NCO-Gruppen eines Polyisocyanates E1) mit einer Isocyanatfunktionalität von 2 bis 6, basierend auf aliphatischen Disocyanaten. Beispielhaft für derartige Polyisocyanate E1) sind Biuret-Strukturen, Isocyanurate bzw. Uretdione basierend auf aliphatischen Diisocyanaten. Dabei werden das Polyisocyanat E1) und das Polyalkylenoxid E2) bevorzugt über eine Urethangruppe bzw. eine Harnstoffgruppe miteinander verknüpft, wobei besonders die Verknüpfung über Urethangruppen bevorzugt ist.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt bei 60 bis 100 °C.

Wurde mit überschüssigem niedermolekularen Diisocyanat gearbeitet, erfolgt bevorzugt anschließend die Entfernung des Überschusses an niedermolekularem, aliphatischen Diisocyanat besonders bevorzugt durch Dünnschichtdestillation.

Vor, während und nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-*tert*-butylkresol oder Tocopherol zugesetzt werden.

Der NCO-Gehalt der hydrophilen Polyisocyanate E) beträgt bevorzugt 0,3 bis 20 Gew.-%, besonders bevorzugt 2 bis 10 Gew.-% und ganz besonders bevorzugt 3 bis 6 Gew.-%.

Beispiele für niedermolekulare, aliphatische Diisocyanate der Komponente E1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind. Besonders bevorzugt sind Butylendiisocyanat, Hexamethylendiisocyanat, Isophorondiisocyanat, ganz besonders bevorzugt Hexamethylendiisocyanat und Isophorondiisocyanat.

Beispiele für höhermolekulare Polyisocyanate E1) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente E1) höhermolekulare Verbindungen mit Biuret-, Iminooxadiazindion,- Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxiden E2) weisen eine OH-Zahl von 10 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden E2) durch Alkoxylierung geeigneter Startermoleküle ist literaturbekannt (z.B. Ullmanns Encyclopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Die monofunktionellen Polyalkylenoxide E2) besitzen typischerweise zahlenmittlere Molekulargewichte von 220 bis 3700 g/mol, bevorzugt von 500 bis 2800 g/mol.

Bevorzugt ist auch, wenn die monofunktionellen Polyalkylenoxide E2) eine OH-Gruppe als isocyanatreaktive Gruppe aufweisen.

Zur Beschleunigung der Urethanbildung kann die erfindungsgemäße Zusammensetzung Katalysatoren F) enthalten. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnacetat, 1,8-Diazabicyclo[5.4.0]undecen-7 (DBU), 1,5-Diazabicyclo[4.3.0]nonen-5 (DBN), 1,4-Diazabicyclo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyl-tetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren F) Bevorzugt ist auch die Verwendung von 1,8-Diazabi-cyclo[5.4.0]undecen-7 (DBU).

In einer besonders bevorzugten Ausführungsform der Erfindung wird ganz auf Katalysatoren verzichtet.

Zur Verbesserung der Schaumbildung, Schaumstabilität oder der Eigenschaften des resultierenden Polyurethan-Schaums können Verbindungen der Komponente G) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere verwendet. Ganz besonders bevorzugt werden EO/PO-Blockcopolymere allein als Komponente G) eingesetzt.

Zudem können zur Verbesserung der Schaumeigenschaften des resultierenden Polyurethan-Schaums Verbindungen der Komponente H) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Typischerweise werden die Komponenten A) bis H) in folgenden Mengen eingesetzt:

100 Gewichtsteile isocyanatfunktionelles Prepolymer A)

0,1 bis 200 Gewichtsteile wässrige Suspension B)

0 bis 30 Gewichtsteile heterocyclische Oligomere C)

0 bis 5 Gewichtteile $C_8$- bis $C_{22}$-Monocarbonsäuren oder deren Ammonium- oder Alkalisalze oder $C_{12}$- bis $C_{44}$-Dicarbonsäuren oder deren Ammonium- oder Alkalisalze oder Alkalimetallsalze schwacher anorganischer Säuren D)

0 bis 250 Gewichtsteilen hydrophiles Polyisocyanat E)

0 bis 1 Gewichtteile Katalysator F)

0 bis 10 Gewichtsteile Tensid G)

0 bis 20 Gewichtsteile Alkohol H)

Bevorzugt werden die Komponenten A) bis H) in folgenden Mengen eingesetzt:

100 Gewichtsteile isocyanatfunktionelles Prepolymer A)

0,1 bis 100 Gewichtsteile wässrige Suspension B)

1 bis 30 Gewichtsteile heterocyclische Oligomere C)

0,01 bis 5 Gewichtteile $C_8$- bis $C_{22}$-Monocarbonsäuren oder deren Ammonium- oder Alkalisalze oder $C_{12}$- bis $C_{44}$-Dicarbonsäuren oder deren Ammonium- oder Alkalisalze oder Alkalimetallsalze schwacher anorganischer Säuren D)

10 bis 100 Gewichtsteilen hydrophiles Polyisocyanat E)

0 bis 1 Gewichtteile Katalysator F)

0 bis 5 Gewichtsteile Tensid G)

0 bis 10 Gewichtsteile Alkohol H)

Besonders bevorzugt werden die Komponenten A) bis H) in folgenden Mengen eingesetzt:

100 Gewichtsteile isocyanatfunktionelles Prepolymere A)

1 bis 60 Gewichtsteile wässrige Suspension B)

5 bis 15 Gewichtsteile heterocyclische Oligomere C)

0,1 bis 1 Gewichtteile $C_8$- bis $C_{22}$-Monocarbonsäuren oder deren Ammonium- oder Alkalisalze oder $C_{12}$- bis $C_{44}$-Dicarbonsäuren oder deren Ammonium- oder Alkalisalze oder Alkalimetallsalze schwacher anorganischer Säuren D)

20 bis 80 Gewichtsteilen hydrophiles Polyisocyanat E)

0 bis 0,5 Gewichtteile Katalysator F)

0 Gewichtsteile Tensid G)

0 Gewichtsteile Alkohol H)

Ein weiterer Gegenstand der vorliegenden Erfindung ist ein Verfahren zur Herstellung eines hydrophilen, aliphatischen Polyurethan-Schaums, bei dem eine erfindungsgemäße Zusammensetzung durch Vermischen der Komponenten hergestellt, aufgeschäumt und ausgehärtet wird.

Die Herstellung der erfindungsgemäßen hydrophilen, aliphatischen Polyurethan-Schäume erfolgt typischerweise durch Mischen der Komponenten A), B), und gegebenenfalls C), D), E), F), G), H) in beliebiger Reihenfolge, Aufschäumen der Mischung und Aushärtung, bevorzugt durch chemische Vernetzung. Bevorzugt werden die Komponenten A), C) und E) miteinander vorvermischt. Die gegebenenfalls einzusetzenden Carboxylate D) und gegebenenfalls die Tenside G) werden bevorzugt in Form ihrer wässrigen Lösungen dem Reaktionsgemisch zugesetzt. Neben dem Wasser, welches über die Komponente B) notwendigerweise eingebracht wird, kann auch weiteres Wasser zugesetzt werden.

Das Aufschäumen kann grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie $C_3$-$C_6$-Alkane, z.B. Butane, *n*-Pentan, *iso*-Pentan, *cyclo*-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind möglich.

Die anschließende Aushärtung erfolgt typischerweise bei Raumtemperatur.

Nach der Aushärtung kann noch vorhandene Restfeuchte gegebenenfalls mit Hilfe üblicher Verfahren wie z.B. durch konvektiven Lufttrocknung oder Mikrowellentrocknung entfernt werden.

Gegenstand der vorliegenden Erfindung ist überdies auch ein nach dem erfindungsgemäßen Verfahren erhältlicher Polyurethan-Schaum, sowie dessen Verwendung als Mittel zur Behandlung von Wunden.

Der Polyurethan-Schaum weist eine poröse, zumindest teilweise offenzellige Struktur mit miteinander kommunizierenden Zellen auf. Die Dichte des Polyurethan-Schaums liegt typischerweise bei 0,01 bis 0,5 g/cm$^3$ (Bestimmung nach DIN 53420).

Das Absorptionsvermögen gegenüber physiologischer Salzlösung beträgt bei dem Polyurethan-Schaum typischerweise 25 bis 150 g pro 100 cm$^2$ bei einem 5 mm dicken Schaum.

Die Messung erfolgt dabei nach folgendem Verfahren: (Bestimmung nach DIN EN 13726-1, Teil 3.2).

Im Vergleich zu anderen hydrophilen Schäumen kann mit dem erfindungsgemäßen Polyurethan-Schaum auch ohne die Verwendung von superabsorbierenden Polymeren eine sehr hohe Absorption von physiologischer Salzlösung erreicht werden. Selbstverständlich ist die Einarbeitung von Superabsorbern aber auch bei dem erfindungsgemäßen Polyurethan-Schaum möglich.

Der Polyurethan-Schaum enthält in der Regel einen nur geringen mit Wasser extrahierbaren Anteil von maximal 5 Gew.%, bevorzugt von maximal 2 Gew.-%, d.h. sie enthalten nur sehr geringe Mengen an chemisch nicht gebundenen Bestandteilen.

Der Polyurethan-Schaum kann zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung, beispielsweise durch Gammabestrahlung, erfolgt. Die Bestrahlung kann dabei gegebenenfalls unter Schutzgasatmosphäre erfolgen. Der erfindungsgemäße Polyurethan-Schaum hat dabei den großen Vorteil, dass er sich bei Bestrahlung, insbesondere bei Bestrahlung mit Gammastrahlen, nicht verfärbt.

Ebenfalls möglich ist die Zugabe, Einarbeitung oder Beschichtung von bzw. mit antimikrobiellen oder biologischen Wirkstoffen, die sich in Bezug auf die Wundheilung und die Vermeidung von Keimbelastungen positiv auswirkt.

Nach der Herstellung kann der Polyurethan-Schaum nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden können. In der Regel werden dazu Blockschäume nach gängigen Methoden auf die gewünschte Dicke geschnitten wodurch flächige Materialien mit einer Dicke von typischerweise von 10 $\mu$m bis 5 cm, bevorzugt von 0,1 mm bis 1 cm, besonders bevorzugt von 0,1 mm bis 6 mm, ganz besonders bevorzugt von 0,2 mm bis 6 mm erhalten werden.

Mit Hilfe geeigneter Gießtechniken können aber auch direkt durch Auftrag und Aufschäumen der erfindungsgemäßen Zusammensetzung auf ein Substrat, z.B. ein gegebenenfalls vorbehandeltes Papier oder Textil, flächige Schäume erhalten werden.

In einer bevorzugten Variante wird dazu die erfindungsgemäße Zusammensetzung mittels eines Rakels auf ein Substrat aufgebracht, wo im Anschluss an das Rakeln das Aufschäumen erfolgt. Die Spalthöhe des Rakels liegt im Allgemeinen im Bereich von 0,2 bis 20 mm, bevorzugt von 0,5 bis 5 und ganz besonders bevorzugt von 0,8 bis 2 mm. Die Filmbreite des zu verwendenden Rakels kann dem jeweiligen Verwendungszweck angepasst werden. Beispiele sind Filmbreiten zwischen 10 und 5000 mm, bevorzugt zwischen 20 und 2000 mm.

Die erfindungsgemäßen Polyurethan-Schäume sind besonders zur Herstellung von Wundauflagen geeignet. Daher ist ein weiterer Gegenstand der Erfindung eine Wundauflage, die einen erfindungsgemäßen Polyurethan-Schaum umfasst.

Gemäß einer besonders bevorzugten Ausführungsform der erfindungsgemäßen Wundauflage weist sie eine Trägerschicht und / oder eine Klebeschicht und / oder eine Abdeckschicht auf.

Geeignete Trägerschichten sind beispielsweise Filme, Folien, Schäume oder Membranen.

Die Trägerschicht kann nach DIN 53333 oder DIN 54101 wasserdampfdurchlässig ausgebildet sein.

Vorzugsweise kann die Trägerschicht thermoplastische Polymere enthalten, etwa in Form einer Beschichtung, oder daraus bestehen. Unter einem thermoplastischen Polymer ist zunächst ein Polymer zu verstehen, das, wenn es in dem für den Werkstoff für Verarbeitung und Anwendung typischen Temperaturbereich wiederholt erwärmt und abgekühlt werden, thermoplastisch bleibt. Unter thermoplastisch wird die Eigenschaft eines Kunststoffes verstanden, in einem für ihn typischen Temperaturbereich wiederholt in der Wärme zu erweichen und beim Abkühlen zu erhärten und im erweichten Zustand wiederholt durch Fließen zum Beispiel als Formteil, Extrudat oder Umformteil zu Halbzeug oder Gegenständen formbar zu sein.

Bevorzugte thermoplastische Polymere sind Polyurethan, Polyethylen, Polypropylen, Polyvinylchlorid, Polystyrol, Polyether, Polyester, Polyamid, Polycarbonat, Polyether-Polyamid-Copolymeren, Polyacrylat, Polymethacrylat und/oder Polymaleat. Vorteilhafterweise sind die thermoplastischen Polymere elastomer. Ganz besonders bevorzugt enthält die Trägerfolie thermoplastisches Polyurethan (TPU) oder besteht daraus. Besonders geeignet sind thermoplastische Polyurethane, die aus der Gruppe umfassend aliphatische Polyesterpolyurethane, aromatische Polyesterpolyurethane, aliphatische Polyetherpolyurethane und/oder aromatische Polyetherpolyurethane ausgewählt sind. Mit diesen Polymeren lassen sich Trägerschichten in der Form atmungsaktiver elastischer Membranfolien erhalten. Diese zeichnen sich durch hohe Flexibilität und Elastizität über einen breiten Temperaturbereich, eine gute Dichtigkeit für flüssiges Wasser bei gleichzeitig hoher Wasserdampfdurchlässigkeit, Geräuscharmut, gute textile Haptik, Wasch- und Reinigungsbeständigkeit, sehr gute chemische und mechanische Beständigkeit und Weichmacherfreiheit aus.

Besonders bevorzugt ist auch eine Trägerschicht, die als Barriere für Keime wirkt und / gegenüber aus der Wunde austretendem Exsudat eine hohe Dichtigkeit bei gleichzeitiger Durchlässigkeit für Wasserdampf aufweist. Dazu kann die Trägerschicht beispielsweise als semipermeable Membran ausgeführt sein.

Weiterhin ist von Vorteil, wenn die Trägerschicht eine Dicke in dem Bereich von $\geq 5\,\mu$m bis $\leq 80\,\mu$m, insbesondere von $\geq 5\,\mu$m bis $\leq 60\,\mu$m und ganz bevorzugt von $\geq 10\,\mu$m bis $\leq 30\,\mu$m und / oder eine Reißdehnung von über 450% aufweist.

Geeignete Klebeschichten sind beispielsweise Haftklebstoffe auf Polyurethan-, Silikon- oder Acrylatbasis.

Beispiele für Polyurethan-basierten Klebstoffe sind hydrophilen Polyurethan-Elastomere, wie sie in der EP 1 923 077 und in der EP 1 815 875 A1 beschrieben sind. Ebenfalls geeignet sind WO 94/07935 bekannten selbstklebenden hydrophilen Polyurethangelschäume..

Geeignete Abdeckschichten enthalten oder bestehen aus Materialen die zu dem Klebstoff der Klebeschicht eine geringe Haftung aufweisen, wenn die mit diesem in Kontakt gebracht werden. Beispiele für solche Abdeckschichten sind Trennpapiere, die mit einer nicht-haftenden Silikon- oder Polyolefinschicht versehen sind.

Die erfindungsgemäßen Wundauflagen können so gestaltet sein, dass der Polyurethan-Schaum bei der Benutzung in direktem oder indirektem Kontakt mit der zu versorgenden Wunde ist. Bevorzugt wird der Polyurethan-Schaum jedoch in direktem Kontakt mit der Wunde eingesetzt, um eine optimale Absorption von Wundflüssigkeit zu gewährleisten.

Beispielhafte Aufbauten von Wundauflagen, in denen der erfindungsgemäße Polyurethan-Schaum eingesetzt werden kann, sind in der EP 1 815 875, EP 1 923 077 oder WO 98/17328 beschrieben.

**Beispiele**

**Methoden:**

Sofern nicht abweichend gekennzeichnet, beziehen sich alle Prozentangaben auf das Gewicht.

Die Bestimmung der Feststoffgehalte erfolgte nach DIN-EN ISO 3251.

Die Bestimmung der Viskositäten erfolgte bei 23 °C und wurde nach DIN 53019 durchgeführt.

Die NCO-Gehalte wurden volumetrisch gemäß DIN-EN ISO 11909 bestimmt.

Zur Bestimmung der Rohdichte wurden zunächst von einem Stück Schaum dessen Breite (B), Höhe (H) und Dicke (D) gemessen und dessen Masse (M) bestimmt. Anschließend wurde die Rohdichte nach der Formel: Rohdichte = M/(B*H*D) berechnet.

Die Volumenquellung wurde bestimmt, indem von einem flächigen Stück Schaum dessen die Breite ($B_{ungequollen}$), Höhe ($H_{usgequollen}$) und Dicke ($D_{ungequollen}$) gemessen, der Schaum bei Raum-temperatur (23 °C) für 2 Minuten komplett in Wasser eingetaucht und anschließend die Breite ($B_{gequollen}$), Höhe ($H_{gequollen}$) und Dicke ($D_{gequollen}$) des gequollenen Schaums gemessen wurde. Die Volumenquellung wurde dann nach folgender Formel berechnet:

$$\text{Volumenquellung (\%)} = (B_{gequollen} * H_{gequollen} * D_{gequollen}) / (B_{ungequollen} * H_{ungequollen} * D_{ungequollen}) * 100$$

Die Bestimmung der "freien Saugleistung" erfolgte durch Absorption physiologischer Salzlösung nach DIN EN 13726-1, Teil 3.2.

Die Bestimmung der mittleren Teilchengrößen (angegeben ist das Zahlenmittel) der Polyurethan-Suspension 1 erfolgte mittels Laserkorrelations-Spektroskopie (Gerät: Malvern Zetasizer 1000, Malver Inst. Limited).

Die Molekulargewichte wurden mittels Gelpermeationschromatographie (GPC) wie folgt bestimmt: Die Kalibrierung erfolgte mit Polystyrol-Standards mit einem Molekulargewichten von Mp 1.000.000 bis 162. Als Eluent wurde Tetrahydrofuran p.A. verwendet. Die folgenden Parameter wurde bei der Doppelmessung eingehalten: Entgasung: Online - Degasser; Durchfluß: 1 ml/Min; Analysenzeit: 45 Minuten; Detektoren: Refraktometer und UV-Detektor; Injektionsvolumen: 100 $\mu$l - 200 $\mu$l. Die Berechnung der Molmassenmittelwerte $M_w$; $M_n$ und $M_p$ sowie der Polydispersität $M_w/M_n$ erfolgte softwaregestützt. Basislinienpunkte und Auswertegrenzen wurden entsprechend der DIN 55672 Teil 1 festgelegt.

Als Rakel wurde der Zehntner Universalapplikator ZUA 2000 mit einer Filmbreite von 200 mm und einer Spalthöhe einstellbar von 0 bis 3 mm eingesetzt (Fa. Zehntner GmbH, Sissach, Schweiz).

**Substanzen und Abkürzungen:**

Diaminosulfonat:     $NH_2$-$CH_2CH_2$-NH-$CH_2CH_2$-$SO_3$Na (45 %ig in Wasser)

Desmophen® C2200:     Polycarbonatpolyol, OH-Zahl 56 mg KOH/g, zahlenmittleres Molekulargewicht 2000 g/mol (BayerMaterialScience AG, Leverkusen, DE)

PolyTHF® 2000:     Polytetramethylenglykolpolyol, OH-Zahl 56 mg KOH/g, zahlenmittleres Molekulargewicht 2000 g/mol (BASF AG, Ludwigshafen, DE)

PolyTHF® 1000: Polytetramethylenglykolpolyol, OH-Zahl 112 mg KOH/g, zahlenmittleres zahlenmittleres Molekulargewicht 1000 g/mol (BASF AG, Ludwigshafen, DE)

Polyether LB 25: monofunktioneller Polyether auf Ethylenoxid-/Propylenoxidbasis, zahlenmittleres Molekulargewicht 2250 g/mol, OH-Zahl 25 mg KOH/g (BayerMaterialScience AG, Leverkusen, DE)

Desmodur® N 3400: Aliphatisches Polyisocyanat (HDI-Uretdion), NCO-Gehalt 21,8 %, Bayer MaterialScience AG, Leverkusen, Deutschland

Desmodur® N 3300: Aliphatisches Polyisocyanat (HDI-Isocyanurat), NCO-Gehalt 21,8 %, Bayer MaterialScience AG, Leverkusen, Deutschland

Bayhydrol AH XP 2741 Wässrige Polyacrylat-Suspension, 41 % Festkörper, Bayer Material-Science AG, Leverkusen, Deutschland

Dispercoll S4510 Wässrige anionische kolloiddisperse Lösung Suspension von amor-phem Silizimdioxid, 50% Festkörper, Teilchengrösse 55 nm, Bayer MaterialScience AG, Leverkusen, Deutschland

Dispercoll S5005 Wässrige anionische kolloiddisperse Lösung Suspension von amorphem Silizimdioxid, 45% Festkörper, Teilchengrösse 30 nm, Bayer MaterialScience AG, Leverkusen, Deutschland

**Herstellung des Polyurethan-Prepolymers 1 (Komponente A))**

Zu einem Gemisch aus 1000 g Hexamethylendiisocyanat (HDI) und 1g Benzoylchlorid wurde bei 80 °C innerhalb von 3 h 1000 g eines Polyalkylenoxids mit einer Molmasse von 4680 g/mol, gestartet auf Glycerin, einem Ethylenoxidgewichtsanteil von 72 % und einen Propylenoxidgewichtsanteil von 28 %, das zuvor bei 100 °C während 6 h bei einem Druck von 0,1 mbar getrocknet wurde, zugetropft und für 12 h nachgerührt. Das überschüssige HDI wurde durch Dünnschichtdestillation bei 130 °C und 0,1 mbar entfernt, wobei die nicht flüchtigen Bestandteile mit 1 g Chlorpropionsäure stabilisiert wurden. Man erhielt ein Prepolymer mit einem NCO-Gehalt von 2,77 % und einer Viskosität von 3500 mPas.

**Herstellung des hydrophilen Polyisocyanates (Komponente E))**

Ein Gemisch aus 282,5 g Desmodur N 3300 und 843,8 g eines Mono-Hydroxy-funktionellen Polyethers auf Ethylenoxid-/Propylenoxidbasis (mit einem Ethylenoxid-Anteil von 80 mol bezogen auf die Gesamtmenge der enthaltenen Oxyalkylengruppen), zahlenmittleres Molekulargewicht 2250 g/mol (OH-Zahl 25 mg KOH/g) wurde in einer Glasapparatur so lang bei 80 °C gerührt, bis der tritrimetrisch ermittelte Gehalt an NCO-Gruppen konstant war. Man erhielt eine Flüssigkeit mit einem NCO-Gehalt von 4,04 % und einer Viskosität von 3330 mPas.

**Herstellung der wässrigen Polyurethan-Suspension (Komponente B))**

1077,2 g PolyTHF® 2000, 409,7 g PolyTHF® 1000, 830,9 g Desmophen® C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120°C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Suspension hatte nachfolgende Eigenschaften:

| | |
|---|---|
| Feststoffgehalt: | 61,6 % |
| Mittlere Teilchengröße: | 528 nm |
| pH (23°C): | 7,5 |

**Herstellung der Polyurethan-Schäume**

Die beiden Isocyanat-Komponenten A) und E) wurden 15 Sekunden bei einer Rührerdrehzahl von 1200 Upm homogenisiert und dann die weiteren Komponenten hinzugefügt. Die Komponenten wurden in den jeweils in der Tabelle 1 angegebenen Mengen eingesetzt. Als Oligomer wurde dabei jeweils Desmodur® N 3400 und als Carboxylat eine 5%ige Lösung von Natriumoleat in Wasser verwendet. Darüber hinaus zugesetztes Wasser ist extra angegeben.

Nach dem Vermischen der Komponenten wurde weitere 10 Sekunden gerührt und die fertige Zusammensetzung schließlich mit einem Rakel (Spalthöhe 1,5 mm) auf ein silikonisiertes Trennpapier aufgebracht. Hier bildete sich der Polyurethan-Schaum aus.

Zur Bestimmung der Volumenquellung wurde der Schaum in kleinere Stücke geschnitten. Die Bestimmung der Rohdichte, Absorption und Volumenquellung erfolgte dann wie im Abschnitt "Methoden" beschrieben.

Tabelle 1

| | Beispiel 1 | Beispiel 2 | Beispiel 3 | Beispiel 4 | Beispiel 5 | Vergleichsbeispiel 6* | Vergleichsbeispiel 7* |
|---|---|---|---|---|---|---|---|
| **Prepolymer A)aus Bsp.1** | 180 g | 180 g | 180 g | 180 g | 180 g | 180 g | 180 g |
| **Oligomer C)** | 25 g | 25 g | 25 g | 25 g | 25 g | 25 g | 25 g |
| **Wässrige Polyurethan lyurethan-Suspension B)** | 25 g | 87,5 g | | | | | |
| **Bayhydrol AH XP 2741 B)** | | | 30 g | | | | |
| **Dispercoll S5005** | | | | 20 g | | | |
| **Dispercoll S4510** | | | | | 80 g | | |
| **Wasser** | | | | | | 10 g | 38 g |
| **Hydrophiles Polyisocyanat E)** | 45 g | 45 g | 45 g | 45 g | 45 g | 45 g | 45 g |
| **Natrium-Oleat E) als 5 Gew.-%ige Lösung in Wasser** | 24 g | 24 g | 24 g | 24 g | 8,5 g | 24 g | 24 g |
| **Eigenschaften** | | | | | | | |
| **Rohdichte [g/1000 cm$^3$]** | 140 | 193 | 193 | 132 | 173 | 131 | 191 |
| **Absorption [g/100 cm$^2$]** | 72 | 55 | 57 | 70 | 61 | 99 | 73 |
| **Volumenquellung (%)** | 97 | 55 | 52 | 53 | 54 | 138 | 171 |

Oligomer : Desmodur N 3400
*) Vergleichsbeispiel ohne wässrige Suspension , Einsatz von Wasser

EP 2 704 754 B1

Die unter Verwendung der erfindungsgemäßen Zusammensetzungen hergestellten Polyurethan-Schäume der Beispiele 1, 2, 3, 4 und 5 wiesen eine sehr gleichmäßige, feine Porenstruktur und eine angenehme, weiche Haptik auf. Darüber hinaus zeigten sie ein hohes Absorptionsvermögen bei gleichzeitig besonders niedriger Volumenquellung. Ein solche Kombination von Eigenschaften ist mit den bekannten Polyurethan-Schäumen nicht zugänglich. Dies zeigt die Gegenüberstellung der erfindungsgemäßen Schäume mit denen der Vergleichsbeispiele 6 und 7, die aus Zusammensetzungen ohne Zusatz einer wässrigen Suspension hergestellt wurden.

**Patentansprüche**

1. Zusammensetzung umfassend

   A) isocyanatfunktionelle Prepolymere erhältlich durch Umsetzung von

   A1) aliphatischen Diisocyanaten mit
   A2) di- bis hexafunktionellen Polyalkylenoxiden mit einem Ethylenoxidanteil von 50 bis 100 mol%, bezogen auf die Gesamtmenge der enthaltenen Oxyalkylengruppen,

   B) eine wässrige Polyurethansuspensionen, eine wässrige Polyacrylatsuspensionen oder wässrige Kieselsole.

2. Zusammensetzung nach Anspruch 1, **dadurch gekennzeichnet, dass** die Diisocyanate aliphatische Diisocyanate mit einer Molmasse von 140 bis 278 g/mol umfassen oder daraus bestehen.

3. Zusammensetzung nach einem der Ansprüche 1 oder 2, **dadurch gekennzeichnet, dass** die Polyalkylenoxide eine OH-Zahl von 22,5 bis 112 aufweisen.

4. Zusammensetzung nach einem der Ansprüche 1 bis 3, **dadurch gekennzeichnet, dass** sie

   C) heterocyclische, 4-Ring- oder 6-Ring-Oligomere von niedermolekularen, aliphatischen Diisocyanaten mit einer Molmasse von 140 bis 278 g/mol

   umfasst.

5. Zusammensetzung nach einem der Ansprüche 1 bis 4, **dadurch gekennzeichnet, dass** sie C8- bis C22-Monocarbonsäuren oder deren Ammonium- oder Alkalisalze oder C12- bis C44-Dicarbonsäuren oder deren Ammonium- oder Alkalisalze oder Alkalimetallsalze schwacher anorganischer Säuren D) umfasst.

6. Zusammensetzung nach einem der Ansprüche 1 bis 5, **dadurch gekennzeichnet, dass** sie

   E) hydrophile Polyisocyanate, erhältlich durch Umsetzung von

   E1) niedermolekularen, aliphatischen Diisocyanaten insbesondere einer Molmasse von 140 bis 278 g/mol und / oder daraus herstellbaren Polyisocyanaten mit einer Isocyanatfunktionalität von 2 bis 6 mit
   E2) monofunktionellen Polyalkylenoxiden insbesondere einer OH-Zahl von 10 bis 250 und einem Ethylenoxidanteil von 50 bis 100 mol%, bezogen auf die Gesamtmenge der enthaltenen Oxyalkylengruppen,

   umfasst.

7. Zusammensetzung nach einem der Ansprüche 1 bis 6, **dadurch gekennzeichnet, dass** die wässrige Polymer-Suspension B) eine wässrige, hydrophilierte Polyurethan-Suspension, bevorzugt eine wässrige, ionisch hydrophilierte Polyurethan-Suspension und ganz besonders bevorzugt eine wässrige, anionisch hydrophilierte Polyurethan-Suspension ist.

8. Zusammensetzung nach Anspruch 7 **dadurch gekennzeichnet, dass** die wässrige, anionisch hydrophilierte Polyurethan-Suspension B) erhältlich ist, indem

   J) isocyanatfunktionelle Prepolymere aus

J1) organischen Polyisocyanaten

J2) polymeren Polyolen insbesondere mit zahlenmittleren Molekulargewichten von 400 bis 8000 g/mol und / oder OH-Funktionalitäten von 1,5 bis 6,

J3) gegebenenfalls hydroxyfunktionellen Verbindungen, insbesondere mit Molekulargewichten von 62 bis 399 g/mol und

J4) gegebenenfalls isocyanatreaktiven, anionischen oder potentiell anionischen und / oder gegebenenfalls nichtionischen Hydrophilierungsmitteln,

hergestellt, die isocyanatfunktionellen Prepolymere J) durch vollständige oder teilweise Umsetzung ihrer freien NCO-Gruppen mit

K1) isocyanatreaktiven, bevorzugt aminofunktionellen, anionischen oder potentiell anionischen Hydrophilierungsmitteln und / oder

K2) gegebenenfalls aminofunktionellen Verbindungen, insbesondere mit Molekulargewichten von 32 bis 400 g/mol,

kettenverlängert und die Prepolymere J) vor während oder nach der Kettenverlängerung in Wasser dispergiert werden, wobei gegebenenfalls enthaltene potentiell anionische Gruppen durch teilweise oder vollständige Umsetzung mit einem Neutralisationsmittel in die anionische Form überführt werden.

9. Zusammensetzung nach einem der Ansprüche 1 bis 8, **dadurch gekennzeichnet, dass** sie 100 Gewichtsteile Prepolymer A), 0,1 bis 200 Gewichtsteile Polymer-Suspension B), 0 bis 30 Gewichtsteile Oligomere C), 0 bis 5 Gewichtteile $C_8$- bis $C_{12}$-Monocarbonsäuren oder deren Ammonium- oder Alkalisalze oder $C_{12}$- bis $C_{44}$-Dicarbonsäuren oder deren Ammonium- oder Alkalisalze D), 0 bis 250 Gewichtsteile hydrophile Polyisocyanate E), 0 bis 1 Gewichtsteile Katalysatoren F), 0 bis 10 Gewichtsteile Tenside G), 0 bis 20 Gewichtsteile Alkohole H) umfasst.

10. Verfahren zur Herstellung eines hydrophilen, aliphatischen Polyurethan-Schaums, bei dem eine Zusammensetzung nach einem der Ansprüche 1 bis 9 durch Vermischen der Komponenten hergestellt, aufgeschäumt und ausgehärtet wird.

11. Polyurethan-Schaum, erhältlich nach dem Verfahren des Anspruchs 10.

12. Polyurethan-Schaum nach Anspruch 11 zur Verwendung als Mittel zur Behandlung von Wunden.

13. Wundauflage umfassend einen Polyurethan-Schaum nach einem der Ansprüche 11 oder 12.

14. Wundauflage nach Anspruch 13, **dadurch gekennzeichnet, dass** sie eine Trägerschicht und / oder eine Klebeschicht, die insbesondere einen Polyurethan-basierten Klebstoff umfassen kann, und / oder eine Abdeckschicht aufweist.

15. Wundauflage nach Anspruch 14, **dadurch gekennzeichnet, dass** der Polyurethan-Schaum zumindest bereichsweise mit der Trägerschicht verbunden ist.

16. Wundauflage nach einem der Ansprüche 14 oder 15, **dadurch gekennzeichnet, dass** die Klebeschicht zumindest bereichsweise mit dem Polyurethan-Schaum und / oder der dem Polyurethan-Schaum zugewandten Seite der Trägerschicht verbunden ist.

17. Wundauflage nach einem der Ansprüche 14 bis 16, **dadurch gekennzeichnet, dass** die Klebeschicht zumindest bereichsweise lösbar mit der Abdeckschicht verbunden ist.

18. Polymersystem, erhältlich durch Vermischen der Komponenten A) und B) und gegebenenfalls C), D), E), F), G), H) einer Zusammensetzung nach einem der Ansprüche 1 bis 9.

**Claims**

1. Composition comprising

A) isocyanate-functional prepolymers obtainable by reaction of

A1) aliphatic diisocyanates with
A2) di- to hexafunctional polyalkylene oxides having an ethylene oxide fraction of 50 to 100 mol%, based on the total amount of oxyalkylene groups present,

B) an aqueous polyurethane suspension, an aqueous polyacrylate suspension or aqueous silica sol.

2. Composition according to Claim 1, **characterized in that** the diisocyanates comprise or consist of aliphatic diisocyanates having a molar mass of 140 to 278 g/mol.

3. Composition according to either Claim 1 or 2, **characterized in that** the polyalkylene oxides have an OH number of 22.5 to 112.

4. Composition according to any of Claims 1 to 3, **characterized in that** it comprises

C) heterocyclic 4-ring or 6-ring oligomers of low molecular weight aliphatic diisocyanates having a molar mass of 140 to 278 g/mol.

5. Composition according to any of Claims 1 to 4, **characterized in that** it comprises C8 to C22 monocarboxylic acids or ammonium or alkali metal salts thereof or C12 to C44 dicarboxylic acids or ammonium or alkali metal salts thereof or alkali metal salts of weak inorganic acids D).

6. Composition according to any of Claims 1 to 5, **characterized in that** it comprises

E) hydrophilic polyisocyanates obtainable by reaction of

E1 low molecular weight aliphatic diisocyanates especially of molar mass 140 to 278 g/mol and/or polyisocyanates obtainable therefrom with an isocyanate functionality of 2 to 6, with
E2) monofunctional polyalkylene oxides especially of OH number 10 to 250 and an ethylene oxide fraction of 50 to 100 mol%, based on the total amount of oxyalkylene groups present.

7. Composition according to any of Claims 1 to 6, **characterized in that** said aqueous polymer suspension B) is an aqueous, hydrophilicized polyurethane suspension, preferably an aqueous, ionically hydrophilicized polyurethane suspension or most preferably an aqueous, anionically hydrophilicized polyurethane suspension.

8. Composition according to Claim 7, **characterized in that** said aqueous, anionically hydrophilicized polyurethane suspension B) is obtainable by preparing

J) isocyanate-functional prepolymers from

J1) organic polyisocyanates,
J2) polymeric polyols especially with number-average molecular weights of 400 to 8000 g/mol and/or OH functionalities of 1.5 to 6,
J3) optionally hydroxyl-functional compounds, especially with molecular weights of 62 to 399 g/mol, and
J4) optionally isocyanate-reactive, anionic or potentially anionic and/or optionally nonionic hydrophilicizing agents,

chain extending said isocyanate-functional prepolymers J) by complete or partial reaction of their free NCO groups with

K1 isocyanate-reactive, preferably amino-functional, anionic or potentially anionic hydrophilicizing agents, and/or
K2) optionally amino-functional compounds, especially with molecular weights of 32 to 400 g/mol,

and dispersing said prepolymers J) in water before, during or after said chain-extending reaction, wherein any potentially anionic groups present are converted into the anionic form by partial or complete reaction with a neutralizing agent.

9. Composition according to any of Claims 1 to 8, **characterized in that** it comprises 100 parts by weight of prepolymer A), 0.1 to 200 parts by weight of polymer suspension B), 0 to 30 parts by weight of oligomers C), 0 to 5 parts by weight of $C_8$ to $C_{12}$ monocarboxylic acids or ammonium or alkali metal salts thereof or $C_{12}$ to $C_{44}$ dicarboxylic acids or ammonium or alkali salts thereof D), 0 to 250 parts by weight of hydrophilic polyisocyanates E), 0 to 1 part by weight of catalysts F), 0 to 10 parts by weight of surfactants G), 0 to 20 parts by weight of alcohols H).

10. Process for producing a hydrophilic aliphatic polyurethane foam, said process comprising a composition according to any of Claims 1 to 9 being produced by mixing the components, foaming the mixture and curing the foamed mixture.

11. Polyurethane foam obtainable by the process of Claim 10.

12. Polyurethane foam according to Claim 11 for use as means for treating wounds.

13. Wound dressing comprising a polyurethane foam according to either Claim 11 or 12.

14. Wound dressing according to Claim 13, **characterized in that** it includes a backing layer and/or an adhesive layer, which may in particular comprise a polyurethane-based adhesive, and/or a covering layer.

15. Wound dressing according to Claim 14, **characterized in that** the polyurethane foam is at least regionally connected to the backing layer.

16. Wound dressing according to either Claim 14 or 15, **characterized in that** the adhesive layer is at least regionally connected to the polyurethane foam and/or to the polyurethane foam side of the backing layer.

17. Wound dressing according to any of Claims 14 to 16, **characterized in that** the adhesive layer is at least regionally releasably connected to the covering layer.

18. Polymer system obtainable by mixing said components A) and B) and optionally C), D), E), F), G), H) of a composition according to any of Claims 1 to 9.

## Revendications

1. Composition comprenant:

   A) des prépolymères à fonction isocyanate, pouvant être obtenus par mise en réaction de

   A1) des diisocyanates aliphatiques avec
   A2) des polyoxydes d'alkylène bi- à hexafonctionnels ayant une proportion d'oxyde d'éthylène de 50 à 100 % en moles, par rapport à la quantité totale de groupes oxyalkylène contenus,

   B) une suspension aqueuse de polyuréthane, une suspension aqueuse de polyacrylate ou des sols de silice aqueux.

2. Composition selon la revendication 1, **caractérisée en ce que** les diisocyanates comprennent des diisocyanates aliphatiques ayant une masse molaire de 140 à 278 g/mol ou en sont constitués.

3. Composition selon l'une quelconque des revendications 1 ou 2, **caractérisée en ce que** les polyoxydes d'alkylène présentent un indice OH de 22,5 à 112.

4. Composition selon l'une quelconque des revendications 1 à 3, **caractérisée en ce qu'**elle comprend

   C) des oligomères hétérocycliques à cycle à 4 ou 6 de diisocyanates aliphatiques de faible masse molaire, ayant une masse molaire de 140 à 278 g/mol.

5. Composition selon l'une quelconque des revendications 1 à 4, **caractérisée en ce qu'**elle comprend des acides monocarboxyliques en C8 à C22 ou leurs sels d'ammonium ou d'alcalis, ou des acides dicarboxyliques en C12 à C44 ou leurs sels d'ammonium ou d'alcalis, ou des sels de métaux alcalins d'acides inorganiques faibles D).

6. Composition selon l'une quelconque des revendications 1 à 5, **caractérisée en ce qu'**elle comprend

E) des polyisocyanates hydrophiles, pouvant être obtenus par mise en réaction de

E1) des diisocyanates aliphatiques de faible masse molaire, notamment d'une masse molaire de 140 à 278 g/mol et/ou des polyisocyanates fabriqués à partir de ceux-ci ayant une fonctionnalité isocyanate de 2 à 6, avec

E2) des polyoxydes d'alkylène monofonctionnels ayant notamment un indice OH de 10 à 250, et une proportion d'oxyde d'éthylène de 50 à 100 % en moles, par rapport à la quantité totale de groupes oxyalkylène contenus.

7. Composition selon l'une quelconque des revendications 1 à 6, **caractérisée en ce que** la suspension aqueuse de polymère B) est une suspension aqueuse de polyuréthane hydrophilisée, de préférence une suspension aqueuse de polyuréthane hydrophilisée ioniquement et de manière tout particulièrement préférée une suspension aqueuse de polyuréthane hydrophilisée anioniquement.

8. Composition selon la revendication 7, **caractérisée en ce que** la suspension aqueuse de polyuréthane hydrophilisée anioniquement B) peut être obtenue par fabrication de

J) des prépolymères à fonction isocyanate de

J1) des polyisocyanates organiques,
J2) des polyols polymères, ayant notamment des poids moléculaires moyens en nombre de 400 à 8 000 g/mol et/ou des fonctionnalités OH de 1,5 à 6,
J3) éventuellement des composés à fonction hydroxy, notamment ayant des poids moléculaires de 62 à 399 g/mol, et
J4) éventuellement des agents d'hydrophilisation réactifs avec les isocyanates, anioniques ou potentiellement anioniques et/ou éventuellement non ioniques,

allongement de chaîne des prépolymères à fonction isocyanate J) par mise en réaction totale ou partielle de leurs groupes NCO libres avec

K1 des agents d'hydrophilisation réactifs avec les isocyanates, de préférence à fonction amino, anioniques ou potentiellement anioniques, et/ou
K2) éventuellement des composés à fonction amino, notamment ayant des poids moléculaires de 32 à 400 g/mol,

et dispersion des prépolymères J) avant, pendant ou après l'allongement de chaîne dans de l'eau, les groupes potentiellement anioniques éventuellement contenus étant transformés en la forme anionique par mise en réaction partielle ou totale avec un agent de neutralisation.

9. Composition selon l'une quelconque des revendications 1 à 8, **caractérisée en ce qu'**elle comprend 100 parties en poids de prépolymère A), 0,1 à 200 parties en poids de suspension de polymère B), 0 à 30 parties en poids d'oligomères C), 0 à 5 parties en poids d'acides monocarboxyliques en $C_8$ à $C_{12}$ ou leurs sels d'ammonium ou d'alcalis, ou d'acides dicarboxyliques en $C_{12}$ à $C_{44}$ ou leurs sels d'ammonium ou d'alcalis D), 0 à 250 parties en poids de polyisocyanates hydrophiles E), 0 à 1 partie en poids de catalyseurs F), 0 à 10 parties en poids de tensioactifs G), 0 à 20 parties en poids d'alcools H).

10. Procédé de fabrication d'une mousse de polyuréthane hydrophile aliphatique, selon lequel une composition selon l'une quelconque des revendications 1 à 9 est fabriquée par mélange des composants, moussée et durcie.

11. Mousse de polyuréthane, pouvant être obtenue par le procédé de la revendication 10.

12. Mousse de polyuréthane selon la revendication 11, destinée à une utilisation en tant qu'agent pour le traitement de blessures.

13. Pansement comprenant une mousse de polyuréthane selon l'une quelconque des revendications 11 ou 12.

**14.** Pansement selon la revendication 13, **caractérisé en ce qu'**il comprend une couche support et/ou une couche adhésive, qui peut notamment comprendre un adhésif à base de polyuréthane, et/ou une couche de recouvrement.

**15.** Pansement selon la revendication 14, **caractérisé en ce que** la mousse de polyuréthane est reliée au moins en partie avec la couche support.

**16.** Pansement selon l'une quelconque des revendications 14 ou 15, **caractérisé en ce que** la couche adhésive est reliée au moins en partie avec la mousse de polyuréthane et/ou le côté orienté vers la couche de polyuréthane de la couche support.

**17.** Pansement selon l'une quelconque des revendications 14 à 16, **caractérisé en ce que** la couche adhésive est reliée au moins en partie de manière amovible avec la couche de recouvrement.

**18.** Système polymère, pouvant être obtenu par mélange des composants A) et B), et éventuellement C), D), E), F), G), H), d'une composition selon l'une quelconque des revendications 1 à 9.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2143744 A1
- DE 2446440 A
- EP 0916647 A
- WO 0188006 A
- WO 0039181 A
- EP 1923077 A
- EP 1815875 A1
- WO 9407935 A
- EP 1815875 A
- WO 9817328 A

**In der Beschreibung aufgeführte Nicht-Patentliteratur**

- Ullmanns Encyclopädie der technischen Chemie. Verlag Chemie, vol. 19, 31-38